# EUROPEAN PATENT SPECIFICATION

(11) **EP 3 471 792 B1**
(45) Date of publication and mention of the grant of the patent: **09.04.2025**
(21) Application number: 17728860.2
(22) Date of filing: 09.06.2017
(51) Int. Cl.: A61M 1/00, A61M 25/00

(54) **PROBE DEVICE FOR INSERTION INTO A BODY OF A PATIENT**
SONDENVORRICHTUNG ZUR EINFÜHRUNG IN EINEN KÖRPER EINES PATIENTEN
DISPOSITIF DE SONDE DESTINÉ À ÊTRE INTRODUIT DANS LE CORPS D'UN PATIENT

(30) Priority: 15.06.2016 DE 102016210653; 30.09.2016 DE 102016219083
(43) Date of publication of application: 24.04.2019
(73) Proprietor: Raumedic AG, 95213 Münchberg (DE); N.V. Nutricia, 2712 HM Zoetermeer (NL)
(72) Inventor: PRESCHER, Jörg, 97729 Ramsthal (DE); WOLKENSTÖRFER, Reinhold, 91077 Neunkirchen (DE); VAN KINDEREN, Sencha, 2562 Den Haag (NL)
(74) Representative: Schmitt-Nilson Schraud Waibel Wohlfrom Patentanwälte Partnerschaft mbB
(86) International application number: PCT/EP2017/064162
(87) International publication number: WO 2017/216068

(56) References cited:
- EP-A1- 0 242 051
- EP-A1- 2 745 828
- EP-A1- 2 745 828
- WO-A1-02/32477
- WO-A1-02/32477
- WO-A1-2014/164655
- WO-A1-2014/164655
- CN-U- 201 840 761
- CN-U- 203 400 329
- CN-U- 205 287 214
- US-A- 4 641 860
- US-A- 4 641 860
- US-A- 4 931 049
- US-A- 5 667 500
- US-A- 5 667 500
- US-A- 5 843 023
- US-A- 5 843 023
- US-A- 5 947 926
- US-A- 5 947 926
- US-A1- 2002 026 209
- US-A1- 2002 026 209
- US-A1- 2003 018 320
- US-A1- 2010 298 812
- US-A1- 2010 298 812
- US-A1- 2013 158 471
- US-A1- 2015 088 100
- US-A1- 2015 088 100

## Description

The invention relates to a probe device for insertion into a body of a patient.

An assembly of this kind in the form of a feeding probe assembly is known from EP 1 744 718 B1. WO 2016/081 893 A1 describes a fluid transfer assembly. US 2010/0 076 406 A1 discloses a coaxial catheter with Y-shaped branch elements. EP 2 231 247 B1 describes a catheter adapter. US 5,947,926 discloses an apparatus and a method for the treatment of the gastrointestinal tract. US 5,843,023 discloses an aspiration needle with a side port. US 2002/0026209 A1 discloses a method and a device for obtaining prostatic material. WO 2014/164 655 A1 discloses a fluid management system. WO 02/32477 A1 discloses a method and a pump apparatus for combined gastro-intestinal feeding and aspiration. US 5,667,500 discloses a medical lavage apparatus and methods. US 2015/0088100 A1 discloses a catheter assembly including a multi-lumen configuration. EP 2 745 828 A1 discloses a feeding tube with an inflatable balloon component. US 4,641,860 discloses a coupling for a flexible tubing. US 2010/0298812 A1 discloses a catheter system. US 2003/0018320 A1 discloses a feeding probe for parenteral feeding of a patient, and its use. EP 0 242 051 A1 discloses an enteral feeding tube assembly and a suction tube therefor. US 2013/0158471 A1 discloses a dual cannula system for enteric feeding. CN 201 840 761 U discloses a multifunctional anti-slip fixed type stomach tube. CN 205 287 214 U discloses a stomach tube for pollution abatement. CN 203 400 329 U discloses a double-port stomach tube. US 4 931 049 A discloses a catheter coupling system.

It is an object of the present invention to improve the versatility of such a device, in particular in the area of a medical application, or an application in which biocompatibility is important.

According to the invention, this object is achieved by the probe device having the features set out in Claim 1.

According to the invention, it has been found possible that requirements placed on a probe device to be inserted into a body of a patient, namely suction on the one hand and feeding or nurturing on the other hand, can be combined with each other. The probe device constitutes both a suction conduit, through which secretions in particular can be aspirated from the body of the patient, and also a feed conduit. The probe device can be configured in form of a catheter. The probe device can be configured in the form of a three-way probe. The probe device can have a Y-shaped configuration. An acute angle of such a Y-shaped configuration can lie between the feed attachment port and the suction attachment port. This acute angle can lie in the range of between 30° and 60°. The probe device can also be made from a plastics material. Components of the probe device can be produced from silicone and/or a silicone-free plastics material, as is explained in more detail below in conjunction with a tube/connector assembly. Alternatively, components of the probe device can also be configured as metal components or metal-containing components, as is likewise explained in detail below in conjunction with the tube/connector assembly. The feed conduit has a feed tube section extending between the feed attachment port and the suction/feed port, wherein the feed tube section has a material composition containing silicone and BaSo₄, wherein the feed attachment port is connected to the feed tube section in a fluid-tight manner via an overmould section which covers a transition between the feed attachment port and the feed tube section and is connected on the one hand in a fluid-tight manner to the feed attachment port and is connected on the other hand in a fluid-tight manner to the feed tube section, wherein the overmould section has a material composition containing silicone and BaSO₄.

In a probe device according to Claim 2, the suctioning function can be provided without the production of a probe unit providing the feeding function having to be greatly modified.

An integrally formed suction tube section according to Claim 3 is cost-effective in terms of production.

A tube transition section according to Claim 4 extends the design options in terms of the configuration of the probe device.

This applies in particular to the use of a three-way connector according to Claim 5. A three-way connector of this kind at the same time constitutes the tube transition section. The three-way connector can also be designed in the form of a three-way valve customary in medical technology. A connection of the three-way connector to the tube sections that are to be attached can be performed in the manner explained below in conjunction with the tube/connector assembly.

An adhesively bonded connection according to Claim 6 is secure. A connection technique of the kind described in WO 2012/163 819 A2 can be used. As an alternative or in addition to an adhesively bonded connection, the tube sections can also be connected by means of overmoulding.

An integrally formed suction tube section according to Claim 7 is cost-effective in terms of production.

An adhesively bonded suction tube section according to Claim 8 again extends the design options for the probe device. Here too, an overmoulding connection can be used alternatively or in addition.

The tube/connector assembly can be in the form of a feeding or nurturing probe assembly for delivering a liquid nutrient to a patient. The tube section can be a tubular tube of a feed probe for predefining a delivery channel section for the liquid nutrient. This delivery channel section can be connected to a source of liquid nutrient. An inner lumen of the tube section can be fluidically connected to the area surrounding the tube section via at least one opening. Such an opening can extend radially in relation to a fluid channel axis or tube axis. Several such openings can be present. In this case, the openings can be arranged axially offset in relation to one another and/or can be mutually offset in the circumferential direction about the tube axis. A high degree of biocompatibility of the assembly is achieved through the silicone content of the tube section. The overmould section ensures a leaktight and in particular tension-resistant connection of the tube section to the tube connector. A surface of the tube connector to be overmoulded can be modified with the aid of a pre-treatment method. By such a pre-treatment method, the adherence, in particular of the overmould section, to the tube connector can be improved. The pre-treatment method can be a corona treatment, a plasma treatment, X-ray sterilization or a post-curing/tempering method. The tube connector can be produced from an in particular silicone-free plastics material. In this case, the tube connector can easily be connected in a fluid-tight manner to a likewise silicone-free external tube. The tube section can have a Shore A hardness in the range of between 50 and 90, in the range of between 60 and 70, and in particular in the region of 70. The plastics material of the tube connector can have a Shore A hardness in the range of between A20 and A90, in the range of between A30 and A60, and in particular in the region of A40. The plastics material of the tube connector can have a modulus of elasticity, in particular a modulus of elasticity in tension, in the range of between 1,000 MPa and 3,000 MPa, and in particular in the range of between 2,000 MPa and 3,000 MPa. The plastics material of the tube connector can be resistant up to a maximum temperature of 80°C, 90°C, 100°C, 110°C, 120°C, 130°C, 140°C, 150°C, 160°C, 170°C or 180°C. The tube connector can alternatively also be produced from silicone or from a non-plastic, for example from stainless steel. The silicone material of the tube section and/or of the overmould section can be a high-temperature vulcanizing silicone (HTV silicone), platinum-crosslinked silicone and/or peroxide-crosslinked silicone. The BaSO₄ fraction of the material composition of the tube section and of the overmould section leads to a stabilization of the connection and can, for example, lead to improved adhesion between the overmould section and the tube connector. Moreover, the BaSO₄ fraction can improve the curing conditions of the component having this fraction. BaSO₄ can moreover be used as X-ray contrast medium.

The advantages of a probe device comprising such a tube-connector assembly correspond to those tubes that have already been explained above with reference to the probe device and the tube/connector assembly.

The overmould section of the tube/connector assembly can have a material composition containing liquid silicone. The use of liquid silicone has been found to be particularly advantageous in the production of the overmould section.

In the area of the transition, an end wall of the tube section of the tube/connector assembly can abut a facing end of the tube connector. An abutting arrangement of this kind permits simple production of the assembly. Alternatively, it is possible to arrange the tube section and the tube connector partially overlapping each other, such that, for example, the tube section is partially pushed onto the connector. An alternative arrangement of this kind can improve the adhesion between the tube section and the connector.

The end of the tube connector of the tube/connector assembly facing towards the transition can have at least one projection via which the tube section bears on the tube connector. Projections of this kind allow material of the overmould section to penetrate between the tube section and the tube connector in the area of the transition, which can lead to stabilization of the connection between these two components and to a tension-resistant connection. The at least one projection can protrude axially from the end face of the tube connector directed towards the transition. At least two such projections can be present. The projections can be integrally formed on a main body of the tube connector. Three, four, five or more projections of this kind can be provided.

The material composition of the tube section can have BaSO₄ in the range of between 2 percent by weight and 90 percent by weight. The material composition of the overmould section can have BaSO₄ in the range of between 2 percent by weight and 90 percent by weight. Such percentages of BaSO₄ have proven to be particularly advantageous for the production of a secure connection between the tube section and the tube connector. The BaSO₄ fractions can be in the range of between 2 percent by weight and 60 percent by weight and in particular between 10 percent by weight and 20 percent by weight.

The tube/connector assembly can have a plug for the distal closure of the tube section, wherein the plug has a material composition containing silicone and BaSO₄. Such a plug closes a distal end of the tube section, such that this end can then be better guided, in particular in a medical application. The material composition of the plug can be the same as that of the overmould section. The material composition of the plug can be the same as that of the tube section.

The material composition of the plug of the tube/connector assembly can have liquid silicone. The material composition of the plug can have 2 percent by weight to 90 percent by weight of BaSO₄. The advantages of such material compositions of the plug correspond to tube that have been explained above in conjunction with the tube section and the overmould section.

The tube connector of the tube/connector assembly can be produced from polybutylene terephthalate (PBT). A tube connector of this kind is stable and non-ageing and, in the case of a medical application, sufficiently biocompatible. Alternatively, the tube connector can be produced, for example, from a cyclo-olefin copolymer (COC), polycarbonate (PC), polyamide (PA), polyphenylene ether (PPE), polyphenylene oxide (PPO), polyphenylene sulphide (PPS) or also PEEK (polyether ether ketone). If the tube connector is produced from plastic, the latter can be a fibre-free or a fibre-reinforced plastic, in particular a glass-fibre-reinforced plastic. In principle, the tube connector can also be produced from a material other than plastic, for example from stainless steel.

Illustrative embodiments of the invention are explained in more detail below with reference to the drawing, in which:
- Fig. 1: shows an interrupted side view of an embodiment of a tube/connector assembly in the form of a probe device designed as a feeding probe assembly for insertion into a body of a patient in order to deliver a liquid nutrient to a patient;
- Fig. 2: shows an axial section through the assembly according to Fig. 1 along line II-II;
- Fig. 3: shows the detail III from Fig. 2;
- Fig. 4: shows the detail IV from Fig. 2;
- Fig. 5: shows an enlarged detail of a tube connector of the assembly, shown in a detail corresponding to Fig. 3;
- Fig. 6: shows a perspective view of the tube connector;
- Fig. 7: shows, in a view similar to Fig. 1, a further embodiment of a tube/connector assembly in the form of a feeding probe assembly;
- Fig. 8: shows a section along line VIII-VIII from Fig. 7;
- Fig. 9: shows the detail IX from Fig. 8;
- Fig. 10: shows the detail X from Fig. 8;
- Fig. 11: shows, in a view similar to Fig. 1, a further embodiment of a tube/connector assembly in the form of a feeding probe assembly;
- Fig. 12: shows a section along line XII-XII from Fig. 11;
- Fig. 13: shows the detail XIII from Fig. 12;
- Fig. 14: shows the detail XIV from Fig. 12;
- Fig. 15: shows, in a view similar to Fig. 1, a further embodiment of a tube/connector assembly in the form of a feeding probe assembly;
- Fig. 16: shows a section along line XVI-XVI from Fig. 15;
- Fig. 17: shows the detail XVII from Fig. 16;
- Fig. 18: shows the detail XVIII from Fig. 16;
- Fig. 19: shows, in a view similar to Fig. 2, a further embodiment of a probe device with a suction conduit configured additionally to a feed conduit;
- Fig. 20: shows, in a view similar to Fig. 2, a further embodiment of a probe device with a suction conduit configured additionally to a feed conduit;
- Fig. 21: shows the detail XXI from Fig. 20;
- Fig. 22: shows, in a view similar to Fig. 2, a further embodiment of a probe device with a suction conduit configured additionally to a feed conduit;
- Fig. 23: shows the detail XXIII from Fig. 22;
- Fig. 24: shows, in a view similar to Fig. 2, a further embodiment of a probe device with a suction conduit configured additionally to a feed conduit;
- Fig. 25: shows the detail XXV from Fig. 24.

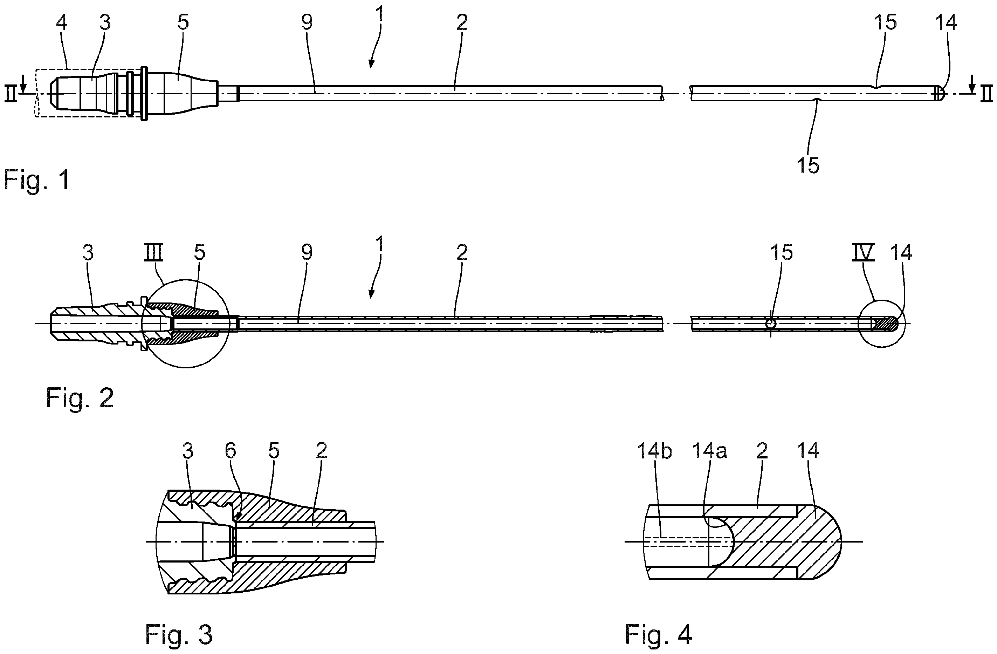

A first embodiment of a tube/connector assembly 1 is explained below with reference to Figures 1 to 6.

This tube/connector assembly 1 is in the form of a feeding probe assembly for delivering a liquid nutrient to a patient. The tube/connector assembly 1 represents a probe device for insertion into a body of a patient. The assembly 1 has a tube section 2 in the form of a tubular tube of the feeding probe. The tube section 2 serves to predefine a delivery channel section of the assembly 1 for the liquid nutrient. The tube section 2 is configured such that it is designed for enteral feeding, in particular for insertion through the nose. A gastric, duodenal or jejunal probe can be formed by means of the tube section. The tube section has a material composition containing silicone and barium sulphate BaSO₄. A Shore A hardness of the tube section 2 can be in the range of between 40 and 90, in the range of between 60 and 80, for example 70. The material composition can contain 2 percent by weight to 60 percent by weight of BaSO₄ and accordingly 40 percent by weight to 98 percent by weight of silicone. In addition to a silicone/BaSO₄ material fraction, other material components can also form the material composition of the tube section 2, in particular further fillers.

The assembly 1 also has a tube connector 3 made of a silicone-free plastics material. The tube connector 3 serves to continue the fluid channel section and to connect the tube section 2 to an external tube 4 indicated by broken lines in Fig. 1. The tube connector 3 is produced from polybutylene terephthalate (PBT). The external tube 4 is in turn fluidically connected to a source of liquid nutrient. The tube connector 3 thus serves to attach a feed source to the probe device 1.

The tube connector 3 is connected to the tube section 2 in a fluid-tight manner via an overmould section 5. The latter covers a transition 6 (cf. Fig. 3) between the tube section 2 and the tube connector 3.

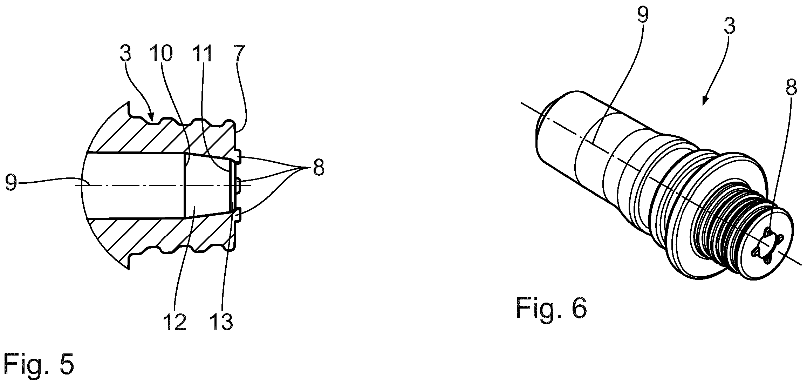

In the assembly 1, the tube section 2 and the tube connector 3 bear on each other end to end at the transition 6. This end-to-end contact is formed on the tube connector 3 by several projections 8 which protrude axially from an end wall 7 and which are integrally formed on a main body of the tube connector 3. An end wall of the tube section 2 facing towards the tube connector 3 thus abuts at least one of the projections 8 in the area of the transition 6. In the embodiment shown, the tube connector 3 has a total of four such projections 8. These are distributed uniformly in the circumferential direction around a fluid channel axis 9 of the assembly 1, as can be seen from the perspective view according to Fig. 6. In the area of the transition 6, an internal diameter of the fluid channel section of the tube connector 3 narrows over several steps 10, 11. A first cone section 12 of the tube connector 3 in this case lies between the narrowing steps 10 and 11. A second cone section 13 is present between the narrowing step 11 and the end wall 7. The cone angles of the cone sections 12 and 13 are different from each other. The cone angle of the second cone section 13 is greater than that of the first cone section 12, such that the fluid channel of the tube connector 3 narrows more strongly in the area of the second cone section 13 than it does in an area of comparable axial extent of the first cone section 12. In the production of the overmould section 5, the second cone section 13 is able to seal off an inner shaping body which predefines the fluid channel and which is pulled back out of the fluid channel after production.

The overmould section 5 is connected in a fluid-tight manner on the one hand to the tube section 2 and on the other hand to the tube connector 3. In the area of the transition 6, material of the overmould section 5 penetrates in the circumferential direction between mutually adjacent projections 8 and thereby ensures stabilizing, possibly also meshing, of the components 2, 3 and 5.

The overmould section 5 has a material composition with silicone and BaSO₄. The silicone can be liquid silicone (LSR - liquid silicone rubber). For the percentages of silicone/BaSO₄ in the material composition of the overmould section 5, and for possible additional material components, reference is made to the details given above concerning the tube section 2.

The assembly 1 moreover has a plug 14 for distal closure of a free end of the tube section 2. The plug 14 also has a material composition containing silicone and BaSO₄. The silicone can be liquid silicone. For the percentages of silicone/BaSO₄ in the material composition of the plug 14, and for possible additional material components, reference is made to the details given above concerning the tube section 2.

A lumen of the tube section 2 is fluidically connected to a surrounding area of the tube section 2 via a plurality of through-openings 15. In an embodiment of the tube section 2 not shown here, there is precisely one through-opening 15. In one embodiment of the tube section 2, there can also be more than two through-openings 15. The through-openings 15 extend radially in relation to the fluid channel axis 9. The through-openings 15 are axially offset with respect to each other. The through-openings 15 are offset in the circumferential direction around the fluid channel axis 9. The one or more through-openings 15 constitute a feed port of the probe device 1.

The plug 14 is rounded at its free end. The complementary end of the plug 14 arranged inside the lumen of the tube section 2 is provided with a concave recess 14a. In the embodiment shown, the recess 14a has the shape of a hollow hemisphere. The recess 14a predefines a bearing position of a guide wire 14b (indicated by broken lines in Fig. 4) for the insertion of the tube section 2. By virtue of the concave design of the recess 14a, a distal end of the guide wire 14b bears securely on the plug 14 and not on an inner wall of the tube section 2.

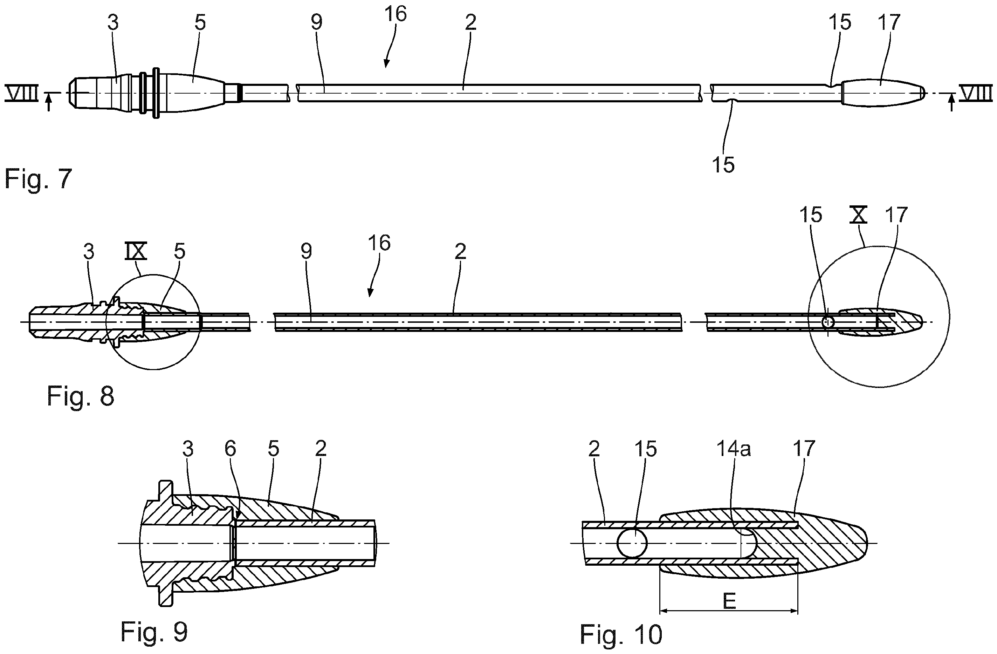

A further embodiment of a tube/connector assembly 16 is explained below with reference to Figures 7 to 10. Components and functions corresponding to tube already explained above with reference to Figures 1 to 6 have the same reference numbers and are not discussed again in detail.

Compared to the assembly 1, the fluid channel in the area of the tube section 2 of the assembly 16 has an enlarged internal diameter. In contrast to the plug 14 of the assembly 1, which does not cover the outer face of the tube section 2, a plug 17 of the assembly 16 does cover a distal end area E of the tube section 2.

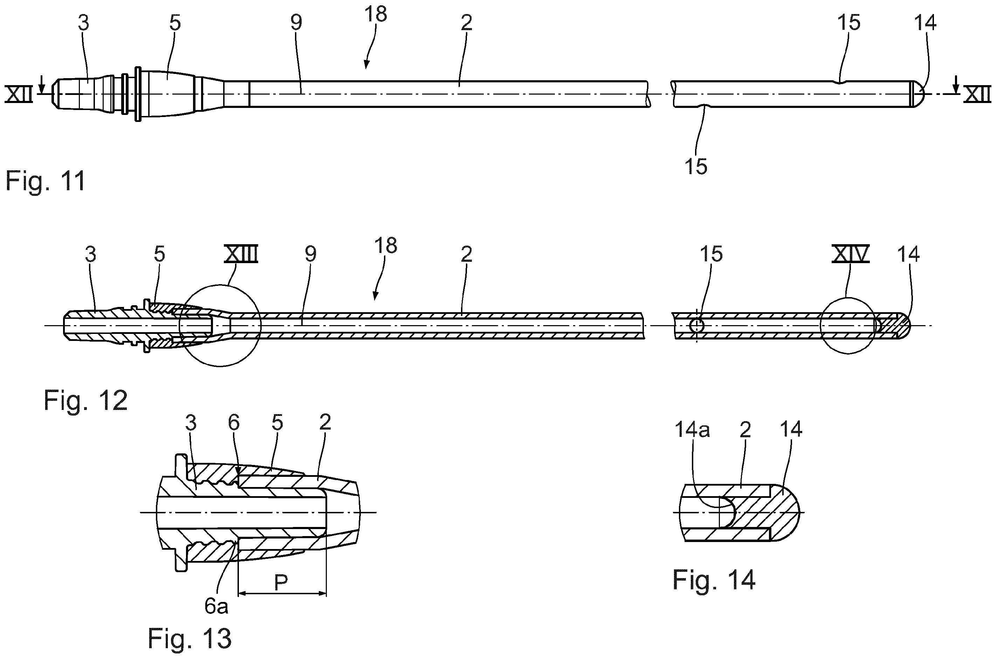

A further embodiment of a tube/connector assembly 18 is explained below with reference to Figures 11 to 14. Components and functions corresponding to tube already explained above with reference to Figures 1 to 10 have the same reference numbers and are not discussed again in detail.

Compared to the assembly 16, the fluid channel in the area of the tube section 2 of the assembly 18 has an enlarged internal diameter. In the assembly 18, the proximal end of the tube section 2 is pushed over the facing end portion of the tube connector 3 in an axial covering area P. In the assembly 18, the transition 6 between the tube connector 3 and the tube section 2 lies at the place where the proximal end wall of the tube section 2 bears on a circumferential rib 6a of the tube connector 3. This transition 6 is again covered by the overmould section 5, wherein, on the one hand, a fluid-tight connection of the overmould section 5 to the tube connector is formed and, on the other hand, a fluid-tight connection of the overmould section 5 to the tube section 2 is formed.

The tube connector 3 of the assembly 18 does not have the projections 8.

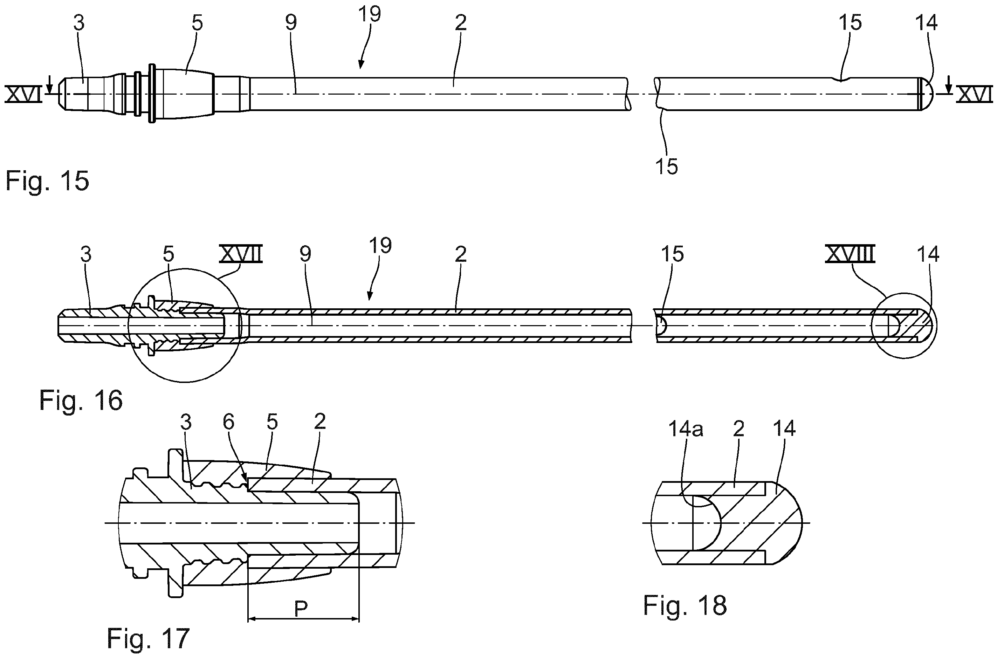

A further embodiment of a tube/connector assembly 19 is explained below with reference to Figures 15 to 18. Components and functions corresponding to tube already explained above with reference to Figures 1 to 14 have the same reference numbers and are not discussed again in detail.

Compared to the assembly 18, the fluid channel in the area of the tube section 2 of the assembly 19 has an enlarged internal diameter.

The transition 6 in the assembly 19, as in the assembly 18, is formed by an end portion pushed proximally onto the tube connector 3.

In the area of the proximal covering P of the assembly 19, an internal diameter of the tube section 2 increases only marginally, in contrast to the embodiment of the assembly 18 where the internal diameter of the tube section 2 increases considerably in the area of the proximal covering P as far as the transition 6.

The overmould section 5 ensures a fluid-tight, stable and sufficiently durable connection between the tube connector 3 and the tube section 2 in all of the described assemblies 1, 16, 18 and 19. A secure connection is thus provided between the silicone-containing tube section 2 and the non-silicone-containing tube connector 3.

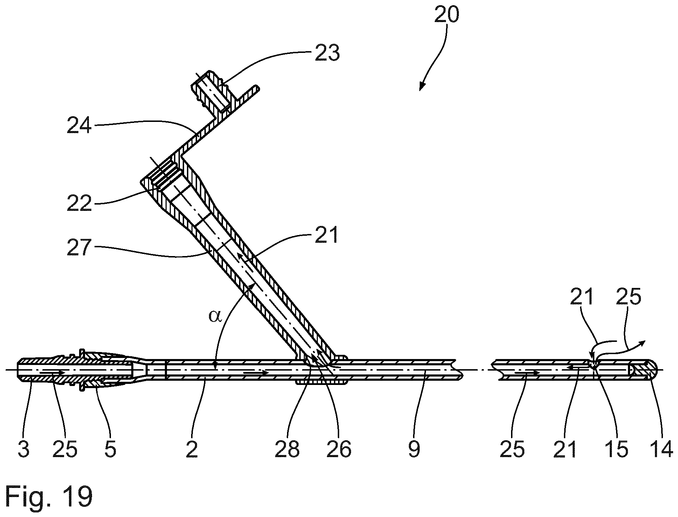

A further embodiment of a probe device 20 is explained below with reference to Figure 19. Components and functions corresponding to tube already explained above with reference to Figures 1 to 18 have the same reference numbers and are not discussed again in detail.

The probe device 20 has a suction conduit 21 between a distal suction/feed port, which is formed by the at least one through opening 15, and a proximal suction attachment port 22 for the attachment of a vacuum source (not shown). The suction attachment port 22 is designed as funnel connector. A closure plug 23 is integrally formed on the suction attachment port 22. The closure plug 23 is connected in one piece to the suction attachment port 22 via a plastic bottle 24.

Moreover, the probe device 20 has a feed conduit 25 between a proximal feed attachment port, formed by the tube connector 3 and serving for the attachment of the feed source, and the distal suction feed port 15.

Overall, therefore, the probe device 20 constitutes a three-way probe which, on the one hand, has a suctioning function via the suction conduit 21 when the suction attachment port 22 is opened and, on the other hand, has a feeding function via the feed conduit 25 when the suction attachment port 22 is closed.

The probe device 20 is present in a Y shape. The feed conduit 25 extends along the tube section 2 without branching off. In the area of an entrance 26 of a suction tube section 27, the suction conduit 21 extends at a bend angle α with respect to the fluid channel axis 9 of the tube section 2. The angle α is an acute angle. The angle α can be in the range of between 30° and 60°.

The tube section 2 is designed as a feed tube section extending between the feed attachment port 3 and the suction/feed port 15. A jacket wall of this tube section 2 has a jacket opening 28 through which the suction conduit 21 opens out from the feed conduit 25.

In the area of the jacket opening 28, i.e. in the area of the entrance point 26, the suction tube section 27 if formed integrally on the feed tube section 2. This can be achieved by injection-moulding the suction tube section 27 onto the feed tube section 2. The suction tube section 27 can be produced from polybutylene terephthalate (PBT) or from silicone. Material variants for the suction tube section 27 are a cyclo-olefin copolymer (COC), polycarbonate (PC), polyamide (PA), polyphenylene ether (PPE), polyphenylene oxide (PPO), polyphenylene sulphide (PPS) or also PEEK (polyether ether ketone).

The probe device 20 is used as follows. If the probe device 20 is to be used to aspirate secretions or other foreign bodies, the closure plug 23 is opened and the vacuum source is attached to the suction attachment port 22. With the probe device 20 correctly inserted, the secretions can then be aspirated through the suction feed port 15 and then flow along the suction conduit 21.

If the feeding function of the probe device 20 is intended to be used, the closure plug 23 is closed and liquid nutrient is delivered to the patient via the feed attachment port 3, i.e. the tube connector, via the feed conduit 25 and the feed port 15, i.e. the at least one through-opening, as has been explained above in conjunction with the embodiments according to Figures 1 to 18.

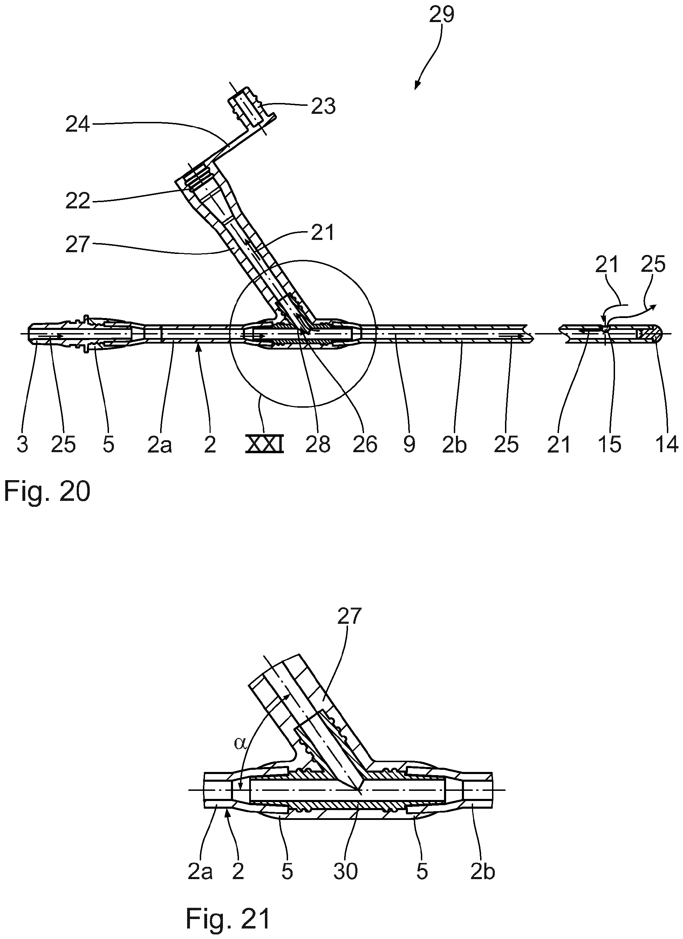

A further embodiment of a probe device 29 is explained below with reference to Figures 20 and 21. Components and functions corresponding to tube already explained above with reference to Figures 1 to 19 have the same reference numbers and are not discussed again in detail.

In the probe device 29, the feed tube section 2 between the feed attachment port 3 and the suction/feed port 15 is divided into two tube sub-sections 2a, 2b. The suction conduit 21 opens out from a tube transition section 30, which is arranged between the two tube sub-sections 2a, 2b, from the feed conduit 25.

The tube transition section 30 is designed as a three-way connector. For fluid transfer, it interconnects a suction/feed tube section, which has the suction/feed port 15, i.e. the tube sub-section 2b, a suction attachment tube section, which has the suction attachment port 22, namely the suction tube section 27, and a feed attachment tube section, which has the feed attachment port 3, namely the tube sub-section 2a. The two tube sub-sections 2a, 2b are connected to the three-way connector 30 via overmould sections 5, as has been described above in particular in conjunction with the embodiments according to Figures 12 and 18. Others of the above-described embodiments of overmould sections can also be used to connect the tube sub-sections 2a, 2b to the three-way connector 30. Together with the tube sub-sections 2a on the one hand and 2b on the other hand, the three-way connector 30 constitutes a tube/connector assembly, as has been explained above with reference to Figures 1 to 18.

The suction tube section 27 is integrally formed in one piece on the overmould section 5, for example by injection moulding. The two overmould sections 5 for the tube sub-sections 2a, 2b are integrally connected to each other.

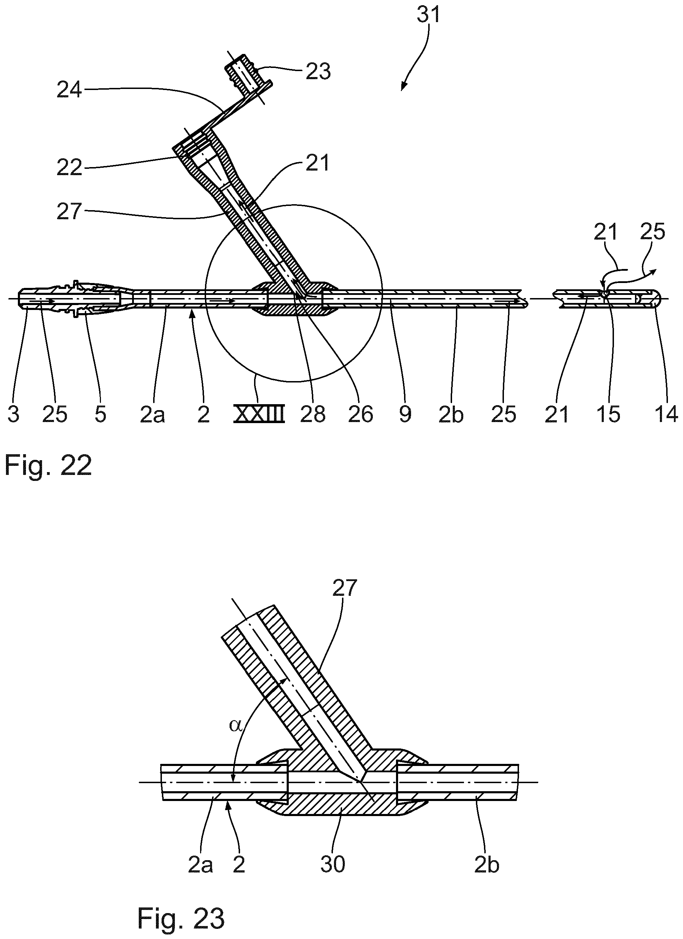

A further embodiment of a probe device 31 is explained below with reference to Figures 22 and 23. Components and functions corresponding to tube already explained above with reference to Figures 1 to 21 have the same reference numbers and are not discussed again in detail.

In the probe device 31, the two tube sub-sections 2a, 2b are each adhesively bonded to the tube transition section 30 of the probe device 31. A connection is used of the type known from WO 2012/163 819 A2. The tube sub-sections 2a, 2b are thus inserted into a main body of the tube transition section 30, wherein the insertion sections inside this main body widen towards the inside via a conically widening inner wall. These conical widenings of the insertion sections extend from the respective insertion opening as far as a constriction step and merge via the latter into a fluid passage of the tube transition section. The conical widening forms an annular space, which can be utilized for the insertion of an adhesive.

In the probe device 31, the suction tube section 27 is formed integrally in one piece on the tube transition section 30.

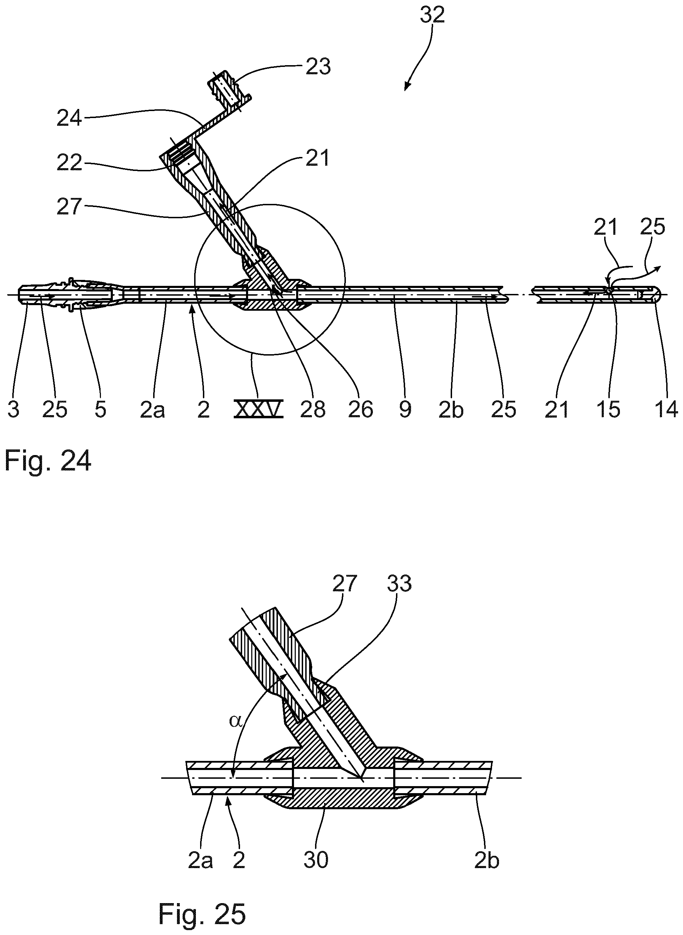

A further embodiment of a probe device 32 is explained below with reference to Figures 24 and 25. Components and functions corresponding to tube already explained above with reference to Figures 1 to 23 have the same reference numbers and are not discussed again in detail.

In contrast to the probe device 31, an adhesively bonded connection of the kind known from WO 2012/163 819 A2 is likewise present between the suction tube section 27 and the tube transition section 30 of the probe device 32. An insertion sub-section 33, via which the suction tube section 27 is inserted into the main body of the tube sub-section 30, has a narrower external diameter compared to the rest of the suction tube section 27.

## Claims

1. Probe device (20; 29; 31; 32) for insertion into a body of a patient
- with a suction conduit (21) between
-- a distal suction/feed port (15) and
-- a proximal suction attachment port (22) for the attachment of a vacuum source,
- with a feed conduit (25) between
-- a proximal feed attachment port (3) for the attachment of a feed source and
-- the distal suction/feed port (15),
wherein the feed conduit (25) has a feed tube section (2) extending between the feed attachment port (3) and the suction/feed port (15), wherein the feed tube section (2) has a material composition containing silicone and BaSO₄,
**characterized in that**
the feed attachment port (3) is connected to the feed tube section (2) in a fluid-tight manner via an overmould section (5) which covers a transition (6) between the feed attachment port (3) and the feed tube section (2) and is connected on the one hand in a fluid-tight manner to the feed attachment port (3) and is connected on the other hand in a fluid-tight manner to the feed tube section (2),
wherein the overmould section (5) has a material composition containing silicone and BaSO4.

2. Probe device according to Claim 1, **characterized in that** a jacket wall of the feed tube section (2) has a jacket opening (28) through which the suction conduit (21) opens out from the feed conduit (25).

3. Probe device according to Claim 2, **characterized in that** a suction tube section (27) in the area of the jacket opening (28) is formed integrally on the feed tube section (2).

4. Probe device according to Claim 1, **characterized in that** the feed conduit (25) has a feed tube section (2) extending between the feed attachment port (3) and the suction/feed port (15) in two tube sub-sections (2a, 2b), wherein the suction conduit (21) opens out from a tube transition section (30) between the two tube sub-sections (2a, 2b).

5. Probe device according to Claim 4, **characterized in that** it comprises a three-way connector by which
- a suction/feed tube section (2b), which has the suction/feed port (15),
- a suction attachment tube section (27), which has the suction attachment port (22), and
- a feed attachment tube section (2a), which has the feed attachment port (3),
are interconnected for fluid transfer.

6. Probe device according to Claim 4 or 5, **characterized in that** the two tube sub-sections (2a, 2b) are adhesively bonded to the tube transition section (30).

7. Probe device according to one of Claims 4 to 6, **characterized in that** a suction tube section (27) is integrally formed on the tube transition section (30).

8. Probe device according to one of Claims 4 to 6, **characterized in that** a suction tube section (27) is adhesively bonded to the tube transition section (30).

## Patentansprüche

1. Sondenvorrichtung (20; 29; 31; 32) zum Einführen in einen Körper eines Patienten
- mit einer Absaugleitung (21) zwischen
-- einem distalen Absaug/Zuführ-Port (15) und
-- einem proximalen Absaug-Anschlussport (22) für den Anschluss einer Vakuumquelle,
- mit einer Zuführleitung (25) zwischen
-- einem proximalen Zuführ-Anschlussport (3) für den Anschluss einer Zuführquelle und
-- dem distalen Absaug/Zuführ-Port (15),
wobei die Zuführleitung (25) einen sich zwischen dem Zuführ-Anschlussport (3) und dem Absaug/Zuführ-Port (15) erstreckenden Zuführ-Schlauch-Abschnitt (2) hat,
wobei der Zuführ-Schlauch-Abschnitt (2) eine Silikon und BaSO₄ enthaltende Materialzusammensetzung hat,
**dadurch gekennzeichnet, dass**
der Zuführ-Anschlussport (3) mit dem Zuführ-Schlauch-Abschnitt (2) in einer fluiddichten Weise über einen Umspritzungsabschnitt (5) verbunden ist, der einen Übergang (6) zwischen dem Zuführ-Anschlussport (3) und dem Zuführ-Schlauch-Abschnitt (2) abdeckt und einerseits in einer fluiddichten Weise mit dem Zuführ-Anschlussport (3) verbunden ist und andererseits in einer fluiddichten Weise mit dem Zuführ-Schlauch-Abschnitt (2) verbunden ist,
wobei der Umspritzungsabschnitt (5) eine Silikon und BaSO₄ enthaltende Materialzusammensetzung hat.

2. Sondenvorrichtung nach Anspruch 1, **dadurch gekennzeichnet, dass** eine Mantelwand des Zuführ-Schlauch-Abschnitts (2) eine Mantelöffnung (28) hat, durch die sich die Absaugleitung (21) aus der Zuführleitung (25) heraus öffnet.

3. Sondenvorrichtung nach Anspruch 2, **dadurch gekennzeichnet, dass** ein Absaug-Schlauch-Abschnitt (27) in dem Bereich der Mantelöffnung (28) integral auf dem Zuführ-Schlauch-Abschnitt (2) ausgebildet ist.

4. Sondenvorrichtung nach Anspruch 1, **dadurch gekennzeichnet, dass** die Zuführleitung (25) einen Zuführ-Leitungsabschnitt (2) hat, der sich zwischen dem Zuführ-Anschlussport (3) und dem Absaug/Zuführ-Port (15) in zwei Schlauch-Unterabschnitten (2a, 2b) erstreckt, wobei sich die Absaugleitung (21) aus einem Schlauch-Übergangs-Abschnitt (30) zwischen den zwei Schlauch-Unterabschnitten (2a, 2b) heraus öffnet.

5. Sondenvorrichtung nach Anspruch 4, **dadurch gekennzeichnet, dass** diese einen Drei-Wege-Verbinder aufweist, durch den
- ein Absaug/Zuführ-Schlauch-Abschnitt (2b), der den Absaug/Zuführ-Port (15) hat,
- ein Absaug-Anschluss-Schlauch-Abschnitt (27), der den Absaug-Anschlussport (22) hat, und
- ein Zuführ-Anschluss-Schlauch-Abschnitt (2a), der den Zuführ-Anschlussport (3) hat,
miteinander für einen Fluidtransfer verbunden sind.

6. Sondenvorrichtung nach Anspruch 4 oder 5, **dadurch gekennzeichnet, dass** die zwei Schlauch-Unterabschnitte (2a, 2b) adhäsiv an dem Schlauch-Übergangs-Abschnitt (30) verbunden sind.

7. Sondenvorrichtung nach einem der Ansprüche 4 bis 6, **dadurch gekennzeichnet, dass** ein Absaug-Schlauch-Abschnitt (27) integral auf dem Schlauch-Übergangs-Abschnitt (30) ausgebildet ist.

8. Sondenvorrichtung nach einem der Ansprüche 4 bis 6, **dadurch gekennzeichnet, dass** ein Absaug-Schlauch-Abschnitt (27) adhäsiv mit dem Schlauch-Übergangs-Abschnitt (30) verbunden ist.

## Revendications

1. Dispositif de sonde (20; 29; 31; 32) pour introduction dans le corps d'un patient
- doté d'un conduit d'aspiration (21) entre
- un orifice d'aspiration/alimentation distal (15), et
- un orifice de fixation d'aspiration proximal (22) pour la fixation d'une source de vide,
- doté d'un conduit d'alimentation (25) entre
- un orifice de fixation d'alimentation proximal (3) pour la fixation d'une source d'alimentation, et
- l'orifice d'aspiration/alimentation distal (15),
dans lequel le conduit d'alimentation (25) présente une section de tube d'alimentation (2) s'étendant entre l'orifice de fixation d'alimentation (3) et l'orifice d'aspiration/alimentation (15) ;
dans lequel la section de tube d'alimentation (2) présente une composition de matériau contenant du silicone et BaSO₄,
**caractérisé en ce que** l'orifice de fixation d'alimentation (3) est raccordé à la section de tube d'alimentation (2) d'une manière étanche aux fluides via une section de surmoulage (5), laquelle recouvre une transition (6) entre l'orifice de fixation d'alimentation (3) et la section de tube d'alimentation (2) et est raccordée, d'une manière étanche aux fluides, d'une part à l'orifice de fixation d'alimentation (3) et est raccordée, d'une manière étanche aux fluides, d'autre part à la section de tube d'alimentation (2), et
dans lequel la section de surmoulage (5) présente une composition de matériau contenant du silicone et BaSO₄.

2. Dispositif de sonde selon la revendication 1, **caractérisé en ce qu'**une paroi de gainage de la section de tube d'alimentation (2) présente une ouverture de gainage (28) à travers laquelle le conduit d'aspiration (21) s'agrandit à partir du conduit d'alimentation (25).

3. Dispositif de sonde selon la revendication 2, **caractérisé en ce qu'**une section de tube d'aspiration (27) dans la zone de l'ouverture de gainage (28) est formée d'un seul tenant sur la section de tube d'alimentation (2).

4. Dispositif de sonde selon la revendication 1, **caractérisé en ce que** le conduit d'alimentation (25) présente une section de tube d'alimentation (2) s'étendant entre l'orifice de fixation d'alimentation (3) et l'orifice d'aspiration/alimentation (15) dans deux sous-sections de tube (2a, 2b), et dans lequel le conduit d'aspiration (21) s'agrandit à partir d'une section de transition de tube (30) entre les deux sous-sections de tube (2a, 2b).

5. Dispositif de sonde selon la revendication 4, **caractérisé en ce qu'**il comprend un raccord à trois voies par lequel
- une section de tube d'aspiration/alimentation (2b), laquelle présente un orifice d'aspiration/alimentation (15) ;
- une section de tube de fixation d'aspiration (27), laquelle présente l'orifice de fixation d'aspiration (22), et
- une section de tube de fixation d'alimentation (2a), laquelle présente l'orifice de fixation d'alimentation (3),
sont interconnectées pour un transfert de fluide.

6. Dispositif de sonde selon la revendication 4 ou 5, **caractérisé en ce que** les deux sous-sections de tube (2a,2b) sont liées de manière adhésive à la section de transition de tube (30).

7. Dispositif de sonde selon l'une quelconque des revendications 4 à 6, **caractérisé en ce qu'**une section de tube d'aspiration (27) est formée d'un seul tenant sur la section de transition de tube (30).

8. Dispositif de sonde selon l'une quelconque des revendications 4 à 6, **caractérisé en ce qu'**une section de tube d'aspiration (27) est collée de manière adhésive sur la section de transition de tube (30).
